# EUROPEAN PATENT SPECIFICATION

(11) **EP 4 469 170 B1**
(45) Date of publication and mention of the grant of the patent: **24.12.2025**
(21) Application number: 23702324.7
(22) Date of filing: 26.01.2023
(51) Int. Cl.: A63B 33/00, A61F 9/02, G02C 3/00

(54) **STRAP ADJUSTMENT MECHANISM**
RIEMENEINSTELLMECHANISMUS
MÉCANISME DE RÉGLAGE DE SANGLE

(30) Priority: 26.01.2022 GB 202201011
(43) Date of publication of application: 04.12.2024
(73) Proprietor: Speedo International Limited, London W1U 3PH (GB)
(72) Inventor: OSMAN, Thomas Frederick, Broadwell Warwickshire CV23 8HF (GB); JOHNSON, Chris, London W1U 3PH (GB); HONG, Jean, London W1U 3PH (GB)
(74) Representative: Mewburn Ellis LLP
(86) International application number: PCT/EP2023/051926
(87) International publication number: WO 2023/144268

(56) References cited:
- EP-A1- 3 012 001
- US-A1- 2009 100 645

## Description

### Technical Field

The present disclosure relates to a strap adjustment mechanism for eyewear, and eyewear including such an adjustment mechanism. The strap adjustment mechanism finds particular, but not exclusive, use in protective eyewear used in water-based activities, such as goggles and snorkelling masks.

### Background

Eyewear is worn for a variety of purposes, including fashion, eye protection, and/or to improve a wearer's vision. Typically, eyewear will include eyepieces (i.e. formed as part of a frame) and a nose bridge that connects the eyepieces.

In some cases, the eyewear will be secured to a user's head by way of arms that project from outer lateral sides of the eyepieces and hook over a user's ears. In other arrangements, a head strap is used to secure the eyewear in place on a user's head. Such arrangements are common in swimming goggles and masks where a tight seal between the eyepieces and a user's face is important to prevent ingress of water into the space therebetween.

Typically, the head strap will be in the form of an elongate band of elastic material that is connected to, and extends between, the outer lateral sides of the eyepieces. In particular, each end of the strap is often looped through an opening formed in a respective eyepiece, such that a central portion of the strap extends from one eyepiece to the other around a wearer's head (when worn).

To ensure a secure and comfortable fit, it is important that the size of the head strap (and thus the tension applied by the head strap) suits a wearer's head size and shape. For this reason, it is known to provide an adjustment mechanism that allows a user to adjust the length of strap extending between the eyepieces (and thus around their head). In some cases, this adjustment mechanism is provided on an eyepiece (usually each eyepiece includes an adjustment mechanism).

One known type of adjustment mechanism includes a component that can be selectively engaged and disengaged with a portion of the strap that loops through the opening in the eyepiece to restrict and allow movement of the strap through the opening. The member can be moved by depressing a button, such that a user is able to disengage the member from the strap to adjust the length of the strap extending between the eyepieces and around the user's head. Such adjustment mechanisms can be difficult to operate (e.g. requiring significant force to be applied by a user) and, in some cases, can cause discomfort to a user when operated while the eyewear is being worn. For example, in some known arrangements, the button for disengaging the strap is arranged such that it is depressed in a direction towards a user's head when worn (usually in the region of a user's temple). As the button is depressed, the mechanism is pushed into a user's head, causing discomfort.

To avoid this discomfort, arrangements have been provided in which buttons are arranged at upper and lower sides of the mechanism, and which are pinched together by a user to disengage a strap. The buttons, when depressed, slide vertically and inwardly towards one another and each engages a respective upper or lower edge of a pawl to lift the pawl away from the strap so as to disengage the strap and allow adjustment. Although this reduces problems of discomfort as discussed above, such a mechanism can be inconvenient to use, because it requires a user to accurately position a finger and a thumb and then apply significant force to effect disengagement.

There is a need to alleviate at least one of the issues described above.

EP3012001A1 proposes an underwater mask including buckle with which a strap can be moved and adjusted in the up-down direction and the lateral direction on a wearer's head.

US2009100645A1 proposes an adjusting device for a goggle strap that is disposed at both ends of the strap for quick connection to two connecting ends of a goggle body.

### Summary

The invention is defined by the appended set of claims.

In a first aspect, there is provided a strap adjustment mechanism for eyewear that includes a strap for securing the eyewear to a user's head, the adjustment mechanism comprising:
a body having an elongate shaft about which a head strap can extend, the elongate extension of the shaft defining a longitudinal direction;
a pawl comprising a mounting portion that movably mounts the pawl to the body and a cam surface facing the body, the pawl extending in a lateral direction towards the shaft from the mounting portion to a free end,
an engagement member moveable between the pawl and the body, the engagement member and cam surface of the pawl configured to cooperate such that lateral movement of the engagement member moves the free end of the pawl between:
   an engaged position in which the free end is able to engage a strap when extending about the shaft; and
   a disengaged position in which the free end is lifted away from the shaft so as to be disengaged from a strap when extending about the shaft.

In such an arrangement, the engagement member moves (e.g. sweeps) between the pawl and the body to lift the pawl away from a strap to disengage the strap. As may be appreciated, such a mechanism can be actuated with a single button press. In this way, the mechanism may be simpler (and more convenient) for a user to operate than, for example, a mechanism that requires multiple buttons to be pressed at the same time

Likewise, the cooperation of the engagement portion and the cam surface is such that a lateral movement of the engagement portion translates into a lifting movement of the pawl. Accordingly, in at least one arrangement of the mechanism, the button can be provided so as to be actuated vertically when worn (avoiding discomfort that could otherwise be caused by actuation in a horizontal direction towards a wearer's head).

The configuration of the cam surface such that it responds to lateral movement (rather than e.g. longitudinal) movement of the engagement arm can provide a more robust mechanism. For example, it means that the engagement member engages the pawl across at least a portion of the width of the pawl, and therefore supports the pawl across at least a portion of its width as it moves the pawl. This helps, for example, to minimise twisting of the pawl as it is actuated (which could otherwise occur with the application of a lifting force to a single edge/side of the pawl).

For the avoidance of doubt, references herein to various components of the mechanism being in the engaged or disengaged position are references to the position of those components when the pawl is in the engaged/disengaged position.

Optional features will now be set out. These are applicable singly or in any combination with any aspect.

The longitudinal direction (or axis), as defined by the extension of the shaft, may be substantially perpendicular to the lateral direction. The pawl may extend from the mounting portion to the free end in a direction that is substantially perpendicular to the shaft.

In an in-use orientation (e.g. when forming part of eyewear worn by a user), the longitudinal direction (or longitudinal axis) may extend substantially vertically. In such an orientation the strap may extend horizontally from the shaft and pass about a wearer's head. In the in-use orientation the lateral direction may be substantially horizontal. A lateral axis (i.e. extending in the lateral direction) may be an in-use forward/rearward axis. Likewise, in the in-use orientation, the free end of the pawl may move in an inward and outward direction towards and away from a user's head (i.e. generally along an inward/outward axis). Thus, when the pawl is lifted away from the shaft, the free end of the pawl may move in an outwardly direction.

The mechanism may comprise a button operatively connected to the engagement member. The operative connection may be configured such that, when depressed, the button causes lateral movement of the engagement member between the pawl and the body. In other words, the button may be moveable between a released position in which the pawl is in the engaged position and a depressed position in which the pawl is in the disengaged position.

The button may be fixedly connected to the engagement member. The button may be integrally formed with the engagement member. The button may comprise a pressing surface configured for pressing by a user to move the button from the released position to the depressed position. The pressing surface may be accessible (i.e. to a user's finger) externally of the body. The pressing surface may be positioned so as to be external of the body. The pressing surface may be proud of an external surface of the body in the engaged position. The button may be at least partly received in a recess formed in the body.

The mounting portion may pivotably mount the pawl to the body. The mounting may be such that the pawl pivots about a pivot axis (e.g. a fixed pivot axis), referred to herein as the pawl pivot axis. The pawl pivot axis may extend longitudinally (e.g. a vertical direction in use). The pawl pivot axis may be substantially parallel to the shaft (i.e. to the elongate extension of the shaft) and may be spaced laterally from the shaft. In the in-use orientation, the pawl pivot axis may be forward of the shaft.

The mounting portion may comprise a pin or recess configured for engagement with a recess or pin of the body. For example, the mounting portion may comprise spaced opposed pins or recesses aligned along the pawl pivot axis for engagement with spaced opposed recesses or pins of the body. Each recess may be in the form of an aperture. In some embodiments, for example, the body may be provided with two projections spaced along the pivot axis, each provided with a recess or a pin for engagement with a corresponding pin or recess of the pawl. The pawl may be configured for snap engagement between the projections.

The engagement member may be arranged to move along a reference plane between the body and the pawl (i.e. the engagement member may move/pivot within a single plane). In the in-use orientation discussed above, the reference plane may be a generally vertical plane and may extend in a substantially forward/rearward direction. In some embodiments, the body may comprise a base surface and the reference plane may be parallel to and/or coplanar to the base surface.

The engagement member may be pivotably mounted to the body. The engagement member may be pivotable about a pivot axis (e.g. a fixed pivot axis), referred to herein is an engagement member pivot axis. The engagement member pivot axis may be substantially perpendicular to the pawl pivot axis. The engagement member pivot axis may be substantially perpendicular to the shaft. The engagement member pivot axis may be substantially perpendicular to the longitudinal and lateral directions. In an in-use orientation (as discussed above) the engagement member pivot axis may extend in the inward/outward direction (i.e. the direction extending towards/away from a user's head).

The engagement member pivot axis may be laterally spaced from the shaft. The engagement member pivot axis may be laterally spaced from the shaft by a greater lateral distance than the mounting portion of the pawl. Thus, the engagement member pivot axis may be further from the shaft than the mounting portion of the pawl.

The engagement member may be interposed between the engagement member pivot axis and the shaft.

The engagement member may be separate to the pawl (i.e. may be or may form part of a component that is not connected to the pawl). The engagement member may be elongate. The engagement member may, for example, extend for a substantial width of the pawl (the width being the dimension of the pawl in the longitudinal direction). The engagement member may extend for more than 30% or 50% or 80% of the width of the pawl. The engagement member may extend for at least the entire width of the pawl (may extend at least entirely across the pawl).

The engagement member may be substantially linear along its length (but in other embodiments may be curved along its length). The engagement member may extend in the longitudinal direction (e.g. may be substantially parallel to the shaft) at least in the engaged position. The engagement member may have a proximal end that is proximate to the button and may extend to a distal end, which may be a free end.

The engagement member may comprise an engagement surface that cooperates with the cam surface (i.e. may be a pawl-facing surface). The engagement surface may be sloped with respect to the direction of movement of the engagement member. That is, the slope of the engagement surface may be oriented so as to extend in the lateral direction. The engagement surface may be sloped relative to the reference plane (and/or the base surface).

The engagement surface maymextend for a substantial width of the pawl (the width being the dimension of the pawl in the longitudinal direction). The engagement surface may extend for more than 30% or 50% or 80% of the width of the pawl. The engagement surface may extend for the entire width of the pawl (may extend entirely across the pawl).

The engagement surface may comprise a leading edge that leads in movement of the engagement member towards the cam surface. The engagement surface may comprise a trailing edge that trails in movement of the engagement member towards the cam surface. The engagement surface may be sloped in a direction from the leading edge to the trailing edge. The engagement surface may slope away from the reference plane (and/or base surface) in a direction from the leading edge to the trailing edge. Thus, the engagement member may be thicker (e.g. in the inward/outward direction) at the trailing edge than the leading edge.

At least a portion of the engagement surface may additionally be sloped in a direction along a length of the engagement member. The engagement surface may slope towards the reference plane in a direction from the proximal end of the engagement member to the distal end. Thus, the engagement member may thickness of the engagement member may taper inwardly in a direction from the proximal end towards the distal end.

The engagement member may comprise proximal and distal portions (i.e. disposed at the proximal and distal ends thereof). The engagement surface may be provided on the proximal portion. Thus, the proximal portion may be tapered (e.g. in both the longitudinal and lateral directions) as discussed above. The distal portion may have a generally continuous cross-sectional shape. When the engagement member is in the disengaged position, the pawl (e.g. the cam surface of the pawl) may rest on the distal portion.

The cam surface of the pawl may be sloped with respect to the direction/axis of movement of the engagement member. That is, the slope of the cam surface may be oriented so as to extend in the lateral direction. The cam surface may be sloped relative to (i.e. may form an angle with) the reference plane (and/or the base surface). The cam surface may be sloped in a direction between the mounting portion and the free end of the pawl.

The cam surface may slope away from the reference plane (and/or base surface) in the direction from the mounting portion to the free end. Thus, the cam surface may face or be directed towards the free end (and the shaft). An end of the cam surface towards the mounting portion may be closer to the reference plane (and/or the body) than an end of the cam surface towards the free end. The cam surface may have a proximal end proximate to the mounting portion and a distal end that is distal from the mounting portion. A distance between the proximal end of the cam surface and the body may be smaller than a distance between the distal end of the cam surface and the body.

The pawl may comprise a limit surface at or proximate to the distal end of the cam surface. The limit surface may extend towards the body (e.g. may be generally perpendicular to the base surface). The limit surface may be arranged to limit movement of the engagement member. The engagement member may abut the limit surface in the engaged position (and may be prevented from moving further towards the shaft by the limit surface).

The free end of the pawl may define a longitudinally extending free edge (i.e. a substantially vertical edge in the in-use orientation) that engages a strap in use. The free edge may be substantially parallel to the shaft. The free end of the pawl may be configured to permit movement of a strap past the pawl in a single (i.e. only one) direction (i.e. restricting movement in the other direction) when in the engaged position. In particular, the free end of the pawl may be configured to permit movement of the strap past the pawl in a direction away from the body (e.g. base surface). On the other hand, the free end of the pawl may restrict/prevent movement of the strap past the pawl in a direction towards the body.

For example, the free end may comprise a tip having a profile that is asymmetrical. That is, the tip may be defined by inner and outer tip surfaces (the inner surface being closer to the body than the outer surface) that join at the free edge and that are asymmetrical. An angle between the inner tip surface and the reference plane may be greater than an angle between the outer surface and the reference plane (the inner surface may be steeper than the outer surface).

The pawl may be biased into the engaged position. The pawl may comprise a pawl biasing portion arranged to urge the pawl into the engaged position. The pawl biasing portion may be resilient (e.g. more resilient than a portion of the pawl extending from the mounting portion to the free end). The pawl biasing portion may comprise a resilient finger. The resilient finger may project from the mounting portion (e.g. laterally) on an opposite side of the mounting portion to the free end of the pawl. The resilient finger may be urged against the body (e.g. a distal end of the resilient finger may contact the body) in at least the disengaged position.

The resilient finger may be curved along its length. The resilient finger may curve towards the body (e.g. may be concave towards the body). The resilient finger may be configured such that movement of the pawl from the engaged position to the disengaged position causes the resilient finger to flex (e.g. may straighten from its curved shape) due to contact with the body. This flexing may bias the pawl out of the disengaged position (i.e. towards the engaged position). The resilient finger may taper inwardly in a direction away from the mounting portion. The pawl (i.e. the biasing portion) may comprise two resilient fingers (each as described above) spaced longitudinally along the mounting portion.

The adjustment mechanism may further comprise a biasing member configured to bias the button and/or engagement member into the engaged position. The provision of a biasing member for the button/engagement member and a separate biasing means for the pawl may increase the ability to control the force required to lift he pawl (i.e. by tuning the two different biasing means). Such control may be desirable so that the force can be tuned, for example, for different user groups (such as for adults and children).

The biasing member may be fixedly connected (e.g. integrally formed with) the engagement member. The biasing member may be resilient (e.g. more resilient than the engagement member). The biasing member may be curved along its length. The biasing member (e.g. a free end of the biasing member) may be urged against the body, at least in the disengaged position. The biasing member may be configured to flex when moved from the engaged position to the disengaged position (due to the contact with the body). The biasing member may extend in the longitudinal direction.

The biasing member may be urged against (so as to contact) a raised abutment portion of the body (e.g. projecting from a base surface of the body) in at least the disengaged position. The raised abutment portion may be in the form of a rib (e.g. extending longitudinally). The biasing member may contact a laterally facing surface of the raised abutment portion. The pawl biasing portion (e.g. the resilient finger(s)) may be urged against (so as to contact) the raised abutment portion in at least the disengaged position to bias the pawl. The pawl biasing portion may contact a distal end surface (e.g. facing outwardly in the in-use orientation) of the raised abutment portion. The biasing member may be positioned between the biasing portion of the pawl biasing portion and the body (e.g. the base surface).

Each of the engagement member, button and biasing member may be fixedly connected (e.g. may be integrally formed). The engagement member, button and biasing member may form at least part of an actuator of the adjustment mechanism. The actuator may comprise an actuator mounting portion pivotably mounting the actuator to the body (i.e. providing the pivotable mounting of the engagement member to the body as described above). The actuator mounting portion may comprise a recess (e.g. aperture) or protrusion for engagement with a corresponding protrusion or recess of the body.

The actuator mounting portion may be connected between the button and the biasing member. That is, the biasing member may project from the actuator mounting portion. Thus, the biasing member may be disposed on an opposite side of the actuator mounting portion to the button.

The actuator mounting portion may be connected to a first lateral end of the button that is opposite to a second lateral end of the button from which the engagement member extends. One or both of the actuator mounting portion and engagement member may extend from/connect to a surface of the button that is opposite to the pressing surface. In this way, the actuator may generally have a U-shaped form, the biasing member and actuator mounting portion forming a first leg of the "U", the engagement member forming the second leg of the "U" and the button joining the first and second legs.

In a second aspect, there is provided a strap adjustment mechanism for eyewear that includes a strap for securing the eyewear to a user's head, the adjustment mechanism comprising:
a body having a longitudinally extending shaft about which a head strap can extend;
a pawl comprising a pawl mounting portion that pivotably mounts the pawl to the body, the pawl extending from the pawl mounting portion to a free end proximate to the shaft,
an actuator comprising an actuator mounting portion that pivotably mounts the actuator to the body, such that the actuator is pivotable along a reference plane extending between the pawl and the body to pivot the pawl between:
   an engaged position in which the free end is able to engage a strap when extending about the shaft; and
a disengaged position in which the free end is lifted away from the shaft so as to be disengaged from the strap.

The adjustment mechanism of the second aspect (e.g. the body, pawl and/or actuator) may be as described above with respect to the first aspect (e.g. may include one or more of the optional features described in the first aspect).

In a third aspect there is provided a strap assembly comprising:
a strap adjustment mechanism as described in the first or second aspect; and
a strap extending about the shaft of the strap adjustment mechanism, the strap comprising a plurality of transverse recesses spaced along at least a portion of the length of the strap, the recesses arranged for engagement with the free end of the pawl of the strap adjustment mechanism.

In a fourth aspect there is provided eyewear comprising first and second eyepieces connected by a nose bridge, at least one of the eyepieces comprising an adjustment mechanism as described in the first or second aspect.

The eyewear may further comprise a strap extending about the shaft of the strap adjustment mechanism. The strap may comprise a plurality of transverse recesses spaced along at least a portion of the length of the strap. The recesses may be arranged for engagement with the free end of the pawl of the strap adjustment mechanism.

### Brief Summary of the Figures

Embodiments will now be discussed with reference to the accompanying figures in which:
Figure 1A is a front view of the interior of a strap adjustment mechanism of a pair of swimming goggles when in an engaged position;
Figure 1B is a bottom view of the strap adjustment mechanism when in the engaged position;
Figure 2A is a front view of the interior of the strap adjustment mechanism when in a disengaged position;
Figure 2B is a bottom view of the strap adjustment mechanism when in the disengaged position;
Figure 3A is a perspective view of a pawl of the strap adjustment mechanism;
Figure 3B is a side view of the pawl;
Figure 4A is a front view of an actuator of the strap adjustment mechanism;
Figure 4B is a rear perspective view of the actuator; and
Figure 5 is a perspective view of a body of the strap adjustment mechanism.

### Detailed Description

Aspects and embodiments will now be discussed with reference to the accompanying figures. Further aspects and embodiments will be apparent to those skilled in the art.

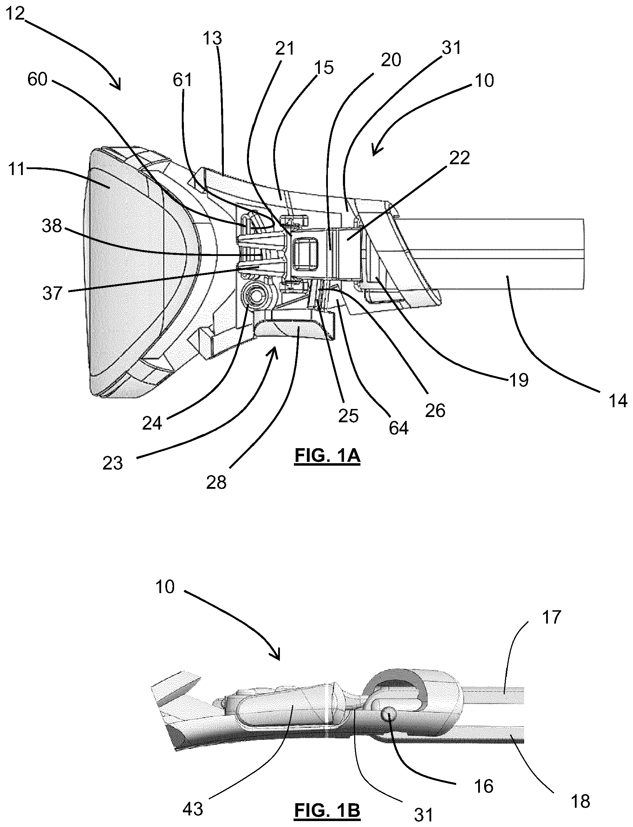

Figures 1A to 2B show a strap adjustment mechanism 10, which forms part of an eyepiece 11 of a pair of swimming goggles 12 (only a portion of which is shown). In particular, the adjustment mechanism 10 is provided at an outer lateral side 13 of the eyepiece 11, at which a head strap 14 (for securing the goggles 12 to a user's head) is attached.

The strap adjustment mechanism 10 includes a body 15 that is integrally formed with the eyepiece 11 (and defines a rearwardly extending arm of the eyepiece 11). The body 15 has a longitudinally extending shaft 16 about which the head strap 14 is looped so as to secure the head strap 14 to the strap adjustment mechanism 10. As should be appreciated, the strap adjustment mechanism 10 further includes a housing (not shown) that mounts to the body 15 so as to enclose the various components shown in the figures and described below.

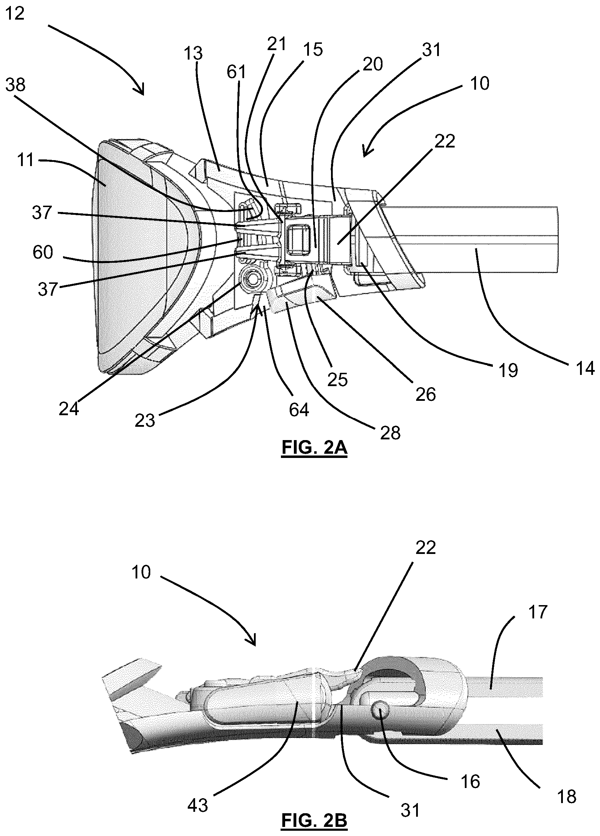

The depiction of the mechanism 10 in Figures 1A and 2A is illustrative of the orientation of the mechanism 10 in normal use (i.e. when worn by a user). In such an orientation, the shaft 16 extends generally vertically (i.e. the longitudinal direction, or a longitudinal axis, is vertical). A lateral direction, as used herein, is horizontal and extends along a generally forward/rearward axis (i.e. forward being in a direction ahead of a user and rearward in a direction behind a user), which is a left/right axis on the page. Likewise, a reference to an inner direction is reference to a direction towards the wearer's head (into the page) and a reference to an outer direction is a reference to a direction away from the wearer's head (out of the page).

Although not shown, a first portion 17 of the strap 14 on one side of the shaft 15 extends to a free end, and a second portion 18 of the strap 14 on the other side of the shaft 15 extends to a further strap adjustment mechanism provided on the other eyepiece of the goggles 12 (i.e. the second portion 17 extending around the back of a user's head in use). The head strap 14 is in the form of an elongate elastic band. One side of the strap 14 is provided with a plurality of transverse recesses 19 (i.e. grooves) that extend across a width of the strap 14 and that are spaced along a portion of the length of the strap 14. As will be described further below, these recesses 19 cooperate with the adjustment mechanism 10 to allow adjustment of the length of the strap 14 extending about a user's head in use.

In particular, the recesses 19 cooperate with a pawl 20 of the adjustment mechanism 10. The pawl 20 comprises a mounting portion 21 that pivotably mounts the pawl 20 to the body 15 such that the pawl 20 pivots about a pawl pivot axis. The pawl pivot axis is parallel to, and forward of, the shaft 16 (vertical and to the left of the shaft 16 as illustrated in Figures 1A and 2A). The pawl 20 extends laterally towards the shaft 16 from the mounting portion 21 to a free end 22 thereof, which engages the recesses 19 of the head strap 14 (in the engaged position as shown in Figures 1A and 1B).

Pivoting of the pawl 20 is effected by an actuator 23 of the strap adjustment mechanism 10. The actuator 23 is pivotally mounted to the body 15 by way of an actuator mounting portion 24. The actuator 23 comprises an elongate engagement member 25 that moves between the pawl 20 and the body 15 (i.e. a substantially planar base surface 31 of the body 15) when the actuator 23 is pivoted. In operation, an engagement surface 26 of the engagement member 25 cooperates with a cam surface 27 on an inner side of the pawl 20 (facing the body 15) such that lateral movement of the engagement member 24 moves the free end 22 of the pawl 20 between an engaged position (as shown in Figures 1A and 1B) and a disengaged position (as shown in Figures 2A and 2B). In the engaged position, the free end 22 of the pawl 20 engages a transverse recess 19 of the strap 14 to restrict movement of the strap 14 about the shaft 16. In the disengaged position the free end 22 of the pawl 20 is lifted away from the strap 14 to permit movement of the strap 14 about the shaft 16.

Accordingly, a user can disengage the pawl 20 to adjust the length of the strap 14 extending between eyepieces 11 (and around their head) and then engage the pawl 20 with the strap 14 to fix the length of strap 14 between the eyepieces 11 (i.e. so that the goggles 12 can be secured to the user's head). The user can bring about such movement of the pawl 20 by pressing a button 28 that forms part of the actuator 23 (and that is therefore operatively connected to the engagement member 25). In particular, pressing the button 28 causes lateral movement of the engagement member 25, such that the engagement surface 26 engages the cam surface 27 and lifts the pawl 20.

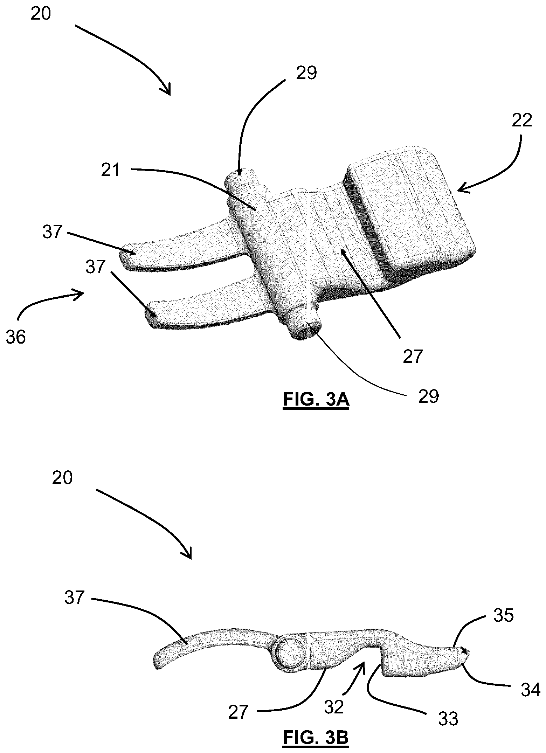

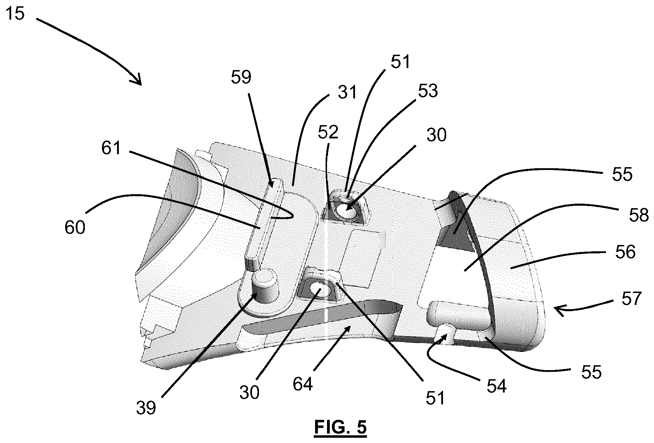

The pawl 20 is shown in greater detail in Figures 3A and 3B. As is apparent from these figures, to provide pivotable mounting the mounting portion 21 of the pawl 20 includes two opposed cylindrical pins 29 that are spaced along the pawl pivot axis (and that project from upper and lower sides of the pawl 20). The pins 29 are received in corresponding circular apertures 30 formed in the body 15 (shown in Figure 5).

The cam surface 27 of the pawl 20 extends from the mounting portion 21 towards the free end 22 of the pawl 20. The cam surface 27 is curved and is sloped with respect to the plane (i.e. reference plane) in which the engagement member 25 moves in use (i.e. the reference plane being parallel to the base surface 31 of the body 15 in the illustrated embodiment). In particular, the engagement member 25 slopes away from the base surface 31 in a direction from the mounting portion 21 to the free end 22 of the pawl 20. Thus, a proximal end of the cam surface 27 (located towards the mounting portion 21) is closer to the base surface 31 of the body 15 than a distal end of the cam surface 21 (located towards the free end 22).

As is particularly evident from Figure 3B, the cam surface 27 partly defines a recess 32 provided in the inner side of the pawl 20. The recess 32 is also partly defined by a limit surface 33 that extends towards the body 15 from the distal end of the cam surface 27. As will be described further below, the engagement member 25 abuts the limit surface 33 when in the engaged position (so as to limit lateral movement of the engagement member 25).

As is also apparent from Figure 3B, the free end 22 of the pawl 20 is configured to permit movement of the head strap 14 past the pawl 20 in a single direction. In particular, the free end 22 has a profile that is asymmetrical. An inner surface 34 of the free end 22 extends on a steeper angle (relative to both the base surface 31 or the reference plane) than an outer surface 35. Accordingly, when the free end 22 of the pawl engages with the strap 14 (specifically, at a transverse recess19 formed in the strap), the asymmetric shape of the free end 22 restricts the strap from being loosened, but allows the strap 14 to be tightened further.

The pawl 20 is configured to be biased into the engaged position. To provide this, the pawl 20 comprises a pawl biasing portion 36 arranged to urge the pawl 20 into the engaged position. The pawl biasing portion 36 comprises two resilient fingers 37 (or prongs) spaced in the longitudinal direction along the mounting portion 21. The resilient fingers 37 project from the mounting portion 21 on an opposite side of the mounting portion 21 to the free end 22 of the pawl 20. The resilient fingers 37 are shaped so as to curve along their respective lengths (so as to be concave towards the body 15), and both fingers 37 taper inwardly in the direction of their extension. As will be described in further detail below, in operation, the resilient fingers 37 contact the body to bias the pawl 20 into the engaged position.

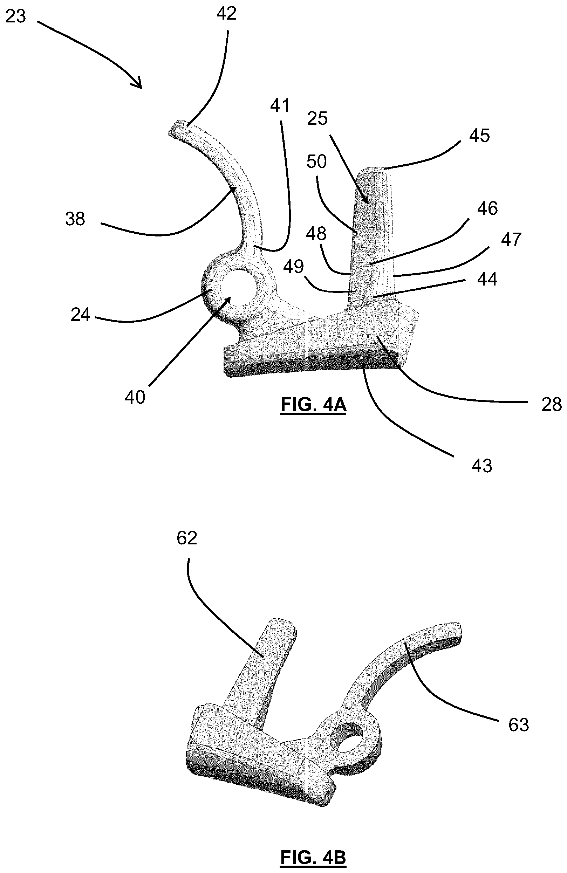

Figures 4A and 4B provide a more detailed view of the actuator 23. The actuator 23 comprises the engagement member 25, the button 28, an elongate biasing member 38, and an actuator mounting portion 24 for pivotably mounting the actuator 23 to the body 15. These components are integrally formed so as to define a unitary structure.

The actuator mounting portion 24 is annular so as to define an aperture 40 for receipt of a corresponding cylindrical protrusion 39 extending outwardly from the base surface 31 of the body 15 (visible in Fig. 5). Accordingly, the actuator 23, and thus the engagement member 25, button 28 and biasing member 38, are pivotable about the protrusion 39 when mounted thereon. In particular, the actuator 23 is pivotable about a pivot axis (referred to herein as an engagement member pivot axis) that extends in an inward/outward direction in the in-use orientation as shown in Figures 1A and 2A. The engagement member pivot axis is substantially perpendicular to the pawl pivot axis and to the direction of extension of the shaft 16.

The biasing member 38 extends in a generally upward direction (in the in-use orientation) from a proximal end 41 adjacent the actuator mounting portion 24 to a distal free end 42 that is distal from the actuator mounting portion 24. The biasing member 38 is arcuate (in the reference plane) and curves in a direction away from the engagement member 25. As will be discussed further below, the biasing member 38 biases the actuator 23 into the engaged position and the arcuate shape of the biasing member facilitates this function.

The button 28 is connected to the actuator mounting portion 24 on an opposite side of the actuator mounting portion 24 to the biasing member 38. Opposite to its connection to the actuator mounting portion 24, the button 28 comprises a pressing surface 43 that (when assembled on the body 15) defines a lower external surface of the mechanism 10 and is therefore able to be pressed by a user (using a finger or thumb) to move the engagement member 25, which in turn moves the pawl 20 from the engaged position to the disengaged position. Specifically, the pressing surface 43 is positioned (and is accessible) at the bottom of the eyepiece 11 (as apparent from Figures 1B and 2B).

Like the actuator mounting portion 24, the engagement member 25 is connected to an upper surface of the button 28 (opposite the pressing surface). The actuator mounting portion 24 is connected to a forward portion of this surface and the engagement member 25 is connected to an opposite, rearward, portion. As a result, the actuator 23 has a generally U-shaped form, with the biasing member 38 and actuator mounting portion 24 forming a first leg of the "U", the engagement member 25 forming the second leg of the "U" and the button 28 forming the base of the "U" joining the first and second legs.

The engagement member 25 extends upwardly from a proximal end 44 (fixed to the button 28) to a distal free end 45 that is distal from the button 28. The engagement member 25 is elongate and substantially linear along its length, and extends in the longitudinal direction in the engaged position.

The engagement member 25 is provided with an engagement surface 46 that cooperates with the cam surface 27 of the pawl 20 (i.e. the engagement surface 46 is a pawl-facing surface). The engagement surface comprises a leading edge 47 that leads in movement of the engagement member 25 towards the cam surface 27 and an opposite trailing edge 48 that trails in movement of the engagement member 25 towards the cam surface 27. The engagement surface 27 is sloped with respect to the base surface 31 (and thus the reference plane in which the engagement member 25 moves) and, in particular, slopes away from the base surface 31 in a direction from the leading edge 47 to the trailing edge 48 (i.e. such that the engagement member 25 is thicker at the trailing edge 48 than at the leading edge 47).

The engagement surface 46 is also sloped in a direction along a length of the engagement member 25. Specifically, a proximal portion 49 of the engagement surface 26 slopes towards the base surface 31 in a direction from the proximal end 44 of the engagement member 25 to the distal end 45. A distal portion 50 of the engagement surface 26...

On an opposite side to the engagement surface 46, the engagement member 25 includes a planar bearing surface 62 that faces, and slides across (in operation), the base surface 31 of the body 15. A similar planar bearing surface 63 is provided on the inward (base surface 31 facing) side of the biasing member 38 and actuator mounting portion 24.

Figure 5 provides a more detailed view of the body 15 of the adjustment mechanism 10. As has been described above, the body 15 defines apertures 30 in which pins 29 of the pawl 20 are received (to provide pivotable mounting). Specifically, the apertures 30 are formed in projections in the form of collars 51 that project from the base surface 31 of the body 15 and that are spaced apart along the pawl pivot axis. An inner surface 52 of each collar 51 (facing the other of the collars 51) includes a guide recess 53 disposed at an end of the collar 51 that is distal from the base surface 31. The guide recesses 53 are configured so as to guide the pins 29 into the apertures 30. In particular, the guide recesses 53 facilitate snap engagement of the pins 29 into the apertures 30. That is, during assembly, the pins 29 are received in the guide recesses 53 and then the mounting portion 21 can be pushed further so as to snap the pins 29 into the apertures 30.

The body 15 also provides means for pivotable mounting of the actuator 23, in the form of a cylindrical protrusion 39 (mentioned briefly above), which is spaced laterally (in the forward direction) from a lower collar 51 of the two collars 51. The cylindrical protrusion 39 projects outwardly from the base surface 31 such that it can be received in the central aperture 40 of the actuator mounting portion 24.

The shaft 16 (not shown in Figure 5) is mounted at a rearward end of the body 15. In particular the shaft 16 is mounted so as to be able to rotate about its longitudinal axis (although in other embodiments it may be fixed). This facilitates movement of the strap 14 about the shaft 16 (i.e. for loosening/tightening of the strap 14). To provide this rotatable mounting, the body 15 includes opposing, axially aligned, bores 54 that receive opposite ends of the shaft 16. Each bore 54 is formed in a respective rearwardly extending arm 55 of the body 15. The arms 55 are spaced longitudinally so as to define an inner opening 58 therebetween, across which the shaft 16 extends (when installed in the bores 54). A cross-member 56 connects the arms 55 (at their rearward ends). The cross-member 56 is arch-shaped so as to define a rearwardly facing opening 57 of the body 15, through which the two strap portions 17, 18 extend when worn.

At an opposite, forward, end of the body 15, the body 15 comprises a raised abutment portion in the form of a rib 59 that projects outwardly from the base surface 31. The rib 59 is elongate in the longitudinal direction (i.e. extends generally parallel to the shaft 16) and includes a distal (outwardly facing) abutment surface 60 and a rearwardly facing lateral abutment surface 61. These abutment surface 60, 61 facilitate biasing of the actuator 23 and the pawl 20. This is best explained with reference to Figures 1A to 2A. As is apparent from these figures, the distal ends of the resilient fingers 37 of the pawl 20 contact the distal abutment surface 60 of the rib 59. When the button 28 is pressed so as to move the engagement member 25 to lift the pawl 20, the pivoting of the pawl 20 and the abutment of the resilient fingers 37 on the rib 59 causes the resilient fingers 37 to flex. This flexing of the fingers 37 is somewhat aided by the curvature of the fingers 37 (as the resilient fingers 37 flex, they straighten). Accordingly, in the disengaged position, the resilient fingers 37 are flexed out of their natural shape, generating internal stresses that result in the fingers 37 urging the pawl 20 back to the disengaged position.

The biasing member 38 operates in a similar manner. The distal end of the biasing member 38 contacts the lateral abutment surface 61 of the rib (such that the biasing member 38 passes underneath the resilient fingers 37). As the button 28 is pressed and the actuator pivots (anti-clockwise, as illustrated) and the biasing member 38 is forced against the lateral abutment surface 61, causing the biasing member 38 to flex (i.e. straighten). Thus, in the disengaged position, the biasing member 38 is flexed out of its natural shape, generating internal stresses that result in the biasing member 38 urging the actuator 23 back towards the engaged position (i.e. urges the actuator 23 in a clockwise direction as illustrated).

As well as ensuring that the engagement member 25 moves rearwardly to allow the pawl 20 to move inwardly, the biasing member 38 also ensures that the button 28 returns to its original position (proud of the lower surface of the body 15). Further movement of the actuator 23 in the clockwise direction (i.e. beyond the engaged position) is prevented by engagement between the engagement member 25 and the limit surface 33 of the pawl 20. Likewise, movement of the actuator in the anti-clockwise direction beyond the disengaged position is limited by engagement of the button 28 at the bottom of a button-receiving recess 64 of the body 15 in which the button 28 is received.

The exemplary embodiments set forth above are considered to be illustrative and not limiting.

For the avoidance of any doubt, any theoretical explanations provided herein are provided for the purposes of improving the understanding of a reader. The inventors do not wish to be bound by any of these theoretical explanations.

Throughout this specification, including the claims which follow, unless the context requires otherwise, the word "comprise" and "include", and variations such as "comprises", "comprising", and "including" will be understood to imply the inclusion of a stated integer or step or group of integers or steps but not the exclusion of any other integer or step or group of integers or steps.

It must be noted that, as used in the specification and the appended claims, the singular forms "a," "an," and "the" include plural referents unless the context clearly dictates otherwise. Ranges may be expressed herein as from "about" one particular value, and/or to "about" another particular value. When such a range is expressed, another embodiment includes from the one particular value and/or to the other particular value. Similarly, when values are expressed as approximations, by the use of the antecedent "about," it will be understood that the particular value forms another embodiment. The term "about" in relation to a numerical value is optional and means for example +/- 10%.

## Claims

1. A strap adjustment mechanism (10) for eyewear (12) that includes a strap (14) for securing the eyewear (12) to a user's head, the adjustment mechanism (10) comprising:
a body (15) having an elongate shaft (16) about which a head strap (14) can extend, the elongate extension of the shaft (16) defining a longitudinal direction;
a pawl (20) comprising a mounting portion (21) that movably mounts the pawl (20) to the body (15) and a cam surface (27) facing the body (15), the pawl (20) extending in a lateral direction towards the shaft (16) from the mounting portion (21) to a free end,
an engagement member (25) moveable between the pawl (20) and the body (15), **characterised in that** the engagement member (25) and cam surface (27) of the pawl (20) are configured to cooperate such that lateral movement of the engagement member (25) moves the free end of the pawl (20) between:
an engaged position in which the free end is able to engage a strap (40) when extending about the shaft (16); and
a disengaged position in which the free end is lifted away from the shaft (16) so as to be disengaged from a strap (40) when extending about the shaft (16).

2. A strap adjustment mechanism (10) according to claim 1 wherein the engagement member (25) comprises an engagement surface (26) that engages the cam surface (27) during lateral movement of the engagement member (25), and wherein the cam surface (27) and/or the engagement surface (26) is sloped relative to a reference plane along which the engagement member (25) moves.

3. A strap adjustment mechanism (10) according to claim 2 wherein the slope(s) of the engagement and/or cam surface(s) (26, 27) are oriented so as to extend in the lateral direction.

4. A strap adjustment mechanism (10) according to any one of the preceding claims wherein the mounting portion (21) pivotably mounts the pawl (20) to the body (15) such that the pawl (20) pivots about a pawl pivot axis.

5. A strap adjustment mechanism (10) according to claim 4 wherein the pawl pivot axis is substantially parallel to the shaft (16).

6. A strap adjustment mechanism (10) according to any one of the preceding claims wherein the pawl (20) comprises a pawl biasing portion (36) arranged to urge the pawl (20) into the engaged position.

7. A strap adjustment mechanism (10) according to claim 6 wherein the pawl biasing portion (36) comprises a resilient finger (37) projecting from the mounting portion (21) on an opposite side of the mounting portion (21) to the free end of the pawl (20).

8. A strap adjustment mechanism (10) according to claim 7 wherein the resilient finger (37) is urged against the body (15) in at least the disengaged position.

9. A strap adjustment mechanism (10) according to any one of the preceding claims wherein the free end of the pawl (20) is configured to permit movement of a strap (14) about the shaft (16) in a single direction when in the engaged position.

10. A strap adjustment mechanism (10) according to any one of the preceding claims wherein the engagement member (25) is pivotably mounted to the body (15) so as to be pivotable about an engagement member pivot axis.

11. A strap adjustment mechanism (10) according to any one of the preceding claims wherein the engagement member (25) is elongate and extends for more than 30% of the width of the pawl (20).

12. A strap adjustment mechanism (10) according to any one of the preceding claims comprising a biasing member (38) configured to bias the engagement member (25) into the engaged position.

13. A strap adjustment mechanism (10) according to any one of the preceding claims comprising a button (28) operatively connected to the engagement member (25), the operative connection configured such that, when depressed, the button (28) causes the lateral movement of the engagement member (25) between the pawl (20) and the body (15).

14. A strap assembly comprising:
a strap adjustment mechanism (10) according to any one of the preceding claims; and
a strap (14) extending about the shaft (16) of the strap adjustment mechanism (10), the strap (14) comprising a plurality of transverse recesses spaced along at least a portion of the length of the strap (14), the recesses arranged for engagement with the free end of the pawl (20) of the strap adjustment mechanism (10).

15. Eyewear (12) comprising first and second eyepieces (11) connected by a nose bridge, at least one of the eyepieces comprising an adjustment mechanism (10) according to any one of claims 1 to 13.

## Patentansprüche

1. Riemeneinstellmechanismus (10) für eine Brille (12), der einen Riemen (14) zum sicheren Fixieren der Brille (12) an dem Kopf eines Benutzers umfasst, wobei der Einstellmechanismus (10) Folgendes umfasst:
einen Körper (15) mit einem länglichen Schaft (16), um den sich ein Kopfriemen (14) erstrecken kann, wobei die längliche Erstreckung des Schafts (16) eine Längsrichtung definiert;
ein Sperrelement (20), umfassend einen Anbringungsabschnitt (21), der das Sperrelement (20) bewegbar an dem Körper (15) anbringt, und eine Nockenfläche (27), die dem Körper (15) zugewandt ist, wobei das Sperrelement (20) sich von dem Anbringungsabschnitt (21) bis zu einem freien Ende in eine seitliche Richtung zum Schaft (16) hin erstreckt,
ein Eingriffselement (25), das zwischen dem Sperrelement (20) und dem Körper (15) bewegbar ist, **dadurch gekennzeichnet, dass** das Eingriffselement (25) und die Nockenfläche (27) des Sperrelements (20) dazu ausgelegt sind, derart zusammenzuwirken, dass die seitliche Bewegung des Eingriffselements (25) das freie Ende des Sperrelements (20) zwischen:
einer in Eingriff gebrachten Position, in der das freie Ende in der Lage ist, mit einem Riemen (40) in Eingriff zu gelangen, wenn er sich um den Schaft (16) erstreckt, und
einer außer Eingriff gebrachten Position, in der das freie Ende von dem Schaft (16) weggehoben ist, um von einem Riemen (40) außer Eingriff gebracht zu sein, wenn er sich um den Schaft (16) erstreckt, bewegt.

2. Riemeneinstellmechanismus (10) nach Anspruch 1, wobei das Eingriffselement (25) eine Eingriffsfläche (26) umfasst, die während einer seitlichen Bewegung des Eingriffselements (25) mit der Nockenfläche (27) in Eingriff gebracht wird, und wobei die Nockenfläche (27) und/oder die Eingriffsfläche (26) relativ zu einer Bezugsebene, entlang derer sich das Eingriffselement (25) bewegt, geneigt ist/sind.

3. Riemeneinstellmechanismus (10) nach Anspruch 2, wobei die Neigung der Eingriffs- und/oder der Nockenfläche (26, 27) derart ausgerichtet ist, dass sie sich in die seitliche Richtung erstreckt.

4. Riemeneinstellmechanismus (10) nach einem der vorangegangenen Ansprüche, wobei der Anbringungsabschnitt (21) das Sperrelement (20) schwenkbar derart an dem Körper (15) anbringt, dass das Sperrelement (20) um eine Sperrelementschwenkachse schwenkt.

5. Riemeneinstellmechanismus (10) nach Anspruch 4, wobei die Sperrelementschwenkachse im Wesentlichen parallel zu dem Schaft (16) ist.

6. Riemeneinstellmechanismus (10) nach einem der vorangegangenen Ansprüche, wobei das Sperrelement (20) einen Sperrelementvorspannabschnitt (36) umfasst, der angeordnet ist, um das Sperrelement (20) in die in Eingriff gebrachte Position zu bewegen.

7. Riemeneinstellmechanismus (10) nach Anspruch 6, wobei der Sperrelementvorspannabschnitt (36) einen nachgiebigen Finger (37) umfasst, der von dem Anbringungsabschnitt (21) auf einer entgegengesetzten Seite des Anbringungsabschnitts (21) bis zum freien Ende des Sperrelements (20) wegsteht.

8. Riemeneinstellmechanismus (10) nach Anspruch 7, wobei der nachgiebige Finger (37) zumindest in der außer Eingriff gebrachten Position gegen den Körper (15) gedrückt wird.

9. Riemeneinstellmechanismus (10) nach einem der vorangegangenen Ansprüche, wobei das freie Ende des Sperrelements (20) dazu ausgelegt ist, eine Bewegung eines Riemens (14) um den Schaft (16) in eine einzige Richtung zu ermöglichen, wenn es sich in der in Eingriff gebrachten Position befindet.

10. Riemeneinstellmechanismus (10) nach einem der vorangegangenen Ansprüche, wobei das Eingriffselement (25) schwenkbar an dem Körper (15) angebracht ist, um um eine Eingriffselementschwenkachse schwenkbar zu sein.

11. Riemeneinstellmechanismus (10) nach einem der vorangegangenen Ansprüche, wobei das Eingriffselement (25) länglich ist und sich über mehr als 30 % der Breite des Sperrelements (20) erstreckt.

12. Riemeneinstellmechanismus (10) nach einem der vorangegangenen Ansprüche, umfassend ein Vorspannelement (38), das dazu ausgelegt ist, das Eingriffselement (25) in die in Eingriff gebrachte Position vorzuspannen.

13. Riemeneinstellmechanismus (10) nach einem der vorangegangenen Ansprüche, umfassend einen Knopf (28), der mit dem Eingriffselement (25) wirkverbunden ist, wobei die Wirkverbindung derart ausgelegt ist, dass der Knopf (28), wenn er heruntergedrückt wird, die seitliche Bewegung des Eingriffselements (25) zwischen dem Sperrelement (20) und dem Körper (15) bewirkt.

14. Riemenanordnung, die Folgendes umfasst:
einen Riemeneinstellmechanismus (10) nach einem der vorangegangenen Ansprüche; und
einen Riemen (14), der sich um den Schaft (16) des Riemeneinstellmechanismus (10) herum erstreckt, wobei der Riemen (14) eine Vielzahl von transversalen Aussparungen umfasst, die entlang von zumindest einem Abschnitt der Länge des Riemens (14) beabstandet sind, wobei die Aussparungen angeordnet sind, um mit dem freien Ende des Sperrelements (20) des Riemeneinstellmechanismus (10) in Eingriff zu gelangen.

15. Brille (12), die ein erstes und ein zweites Augenteil (11), welche durch einen Nasensteg verbunden sind, umfasst, wobei zumindest eines aus den Augenteilen einen Einstellmechanismus (10) nach einem der Ansprüche 1 bis 13 umfasst.

## Revendications

1. Mécanisme d'ajustement de sangle (10) pour lunettes (12) qui inclut une sangle (14) pour fixer les lunettes (12) à la tête d'un utilisateur, le mécanisme d'ajustement (10) comprenant :
un corps (15) présentant un arbre allongé (16) autour duquel une sangle de tête (14) peut s'étendre, l'extension allongée de l'arbre (16) définissant une direction longitudinale ;
un cliquet (20) comprenant une partie de montage (21) qui monte de manière mobile le cliquet (20) sur le corps (15) et une surface de came (27) faisant face au corps (15), le cliquet (20) s'étendant dans une direction latérale vers l'arbre (16) depuis la partie de montage (21) jusqu'à une extrémité libre,
un élément de mise en prise (25) mobile entre le cliquet (20) et le corps (15), **caractérisé en ce que** l'élément de mise en prise (25) et la surface de came (27) du cliquet (20) sont configurés pour coopérer de telle sorte qu'un déplacement latéral de l'élément de mise en prise (25) déplace l'extrémité libre du cliquet (20) entre :
une position en prise dans laquelle l'extrémité libre est capable de venir en prise avec une sangle (40) lorsqu'elle s'étend autour de l'arbre (16) ; et
une position libérée dans laquelle l'extrémité libre est soulevée à l'écart de l'arbre (16) de manière à être libérée d'une sangle (40) lorsqu'elle s'étend autour de l'arbre (16).

2. Mécanisme d'ajustement de sangle (10) selon la revendication 1, dans lequel l'élément de mise en prise (25) comprend une surface de mise en prise (26) qui vient en prise avec la surface de came (27) pendant un déplacement latéral de l'élément de mise en prise (25), et dans lequel la surface de came (27) et/ou la surface de mise en prise (26) est inclinée par rapport à un plan de référence le long duquel l'élément de mise en prise (25) se déplace.

3. Mécanisme d'ajustement de sangle (10) selon la revendication 2, dans lequel la ou les pentes de la ou des surfaces de mise en prise et/ou de came (26, 27) sont orientées de manière à s'étendre dans la direction latérale.

4. Mécanisme d'ajustement de sangle (10) selon l'une quelconque des revendications précédentes, dans lequel la partie de montage (21) monte de manière pivotante le cliquet (20) sur le corps (15) de sorte que le cliquet (20) pivote autour d'un axe de pivotement de cliquet.

5. Mécanisme d'ajustement de sangle (10) selon la revendication 4, dans lequel l'axe de pivotement du cliquet est sensiblement parallèle à l'arbre (16).

6. Mécanisme d'ajustement de sangle (10) selon l'une quelconque des revendications précédentes, dans lequel le cliquet (20) comprend une partie de sollicitation de cliquet (36) agencée pour pousser le cliquet (20) jusque dans la position en prise.

7. Mécanisme d'ajustement de sangle (10) selon la revendication 6, dans lequel la partie de sollicitation de cliquet (36) comprend un doigt élastique (37) faisant saillie à partir de la partie de montage (21) sur un côté opposé de la partie de montage (21) vers l'extrémité libre du cliquet (20).

8. Mécanisme d'ajustement de sangle (10) selon la revendication 7, dans lequel le doigt élastique (37) est poussé contre le corps (15) au moins dans la position libérée.

9. Mécanisme d'ajustement de sangle (10) selon l'une quelconque des revendications précédentes, dans lequel l'extrémité libre du cliquet (20) est configurée pour permettre le déplacement d'une sangle (14) autour de l'arbre (16) dans une seule direction lorsqu'elle est dans la position en prise.

10. Mécanisme d'ajustement de sangle (10) selon l'une quelconque des revendications précédentes, dans lequel l'élément de mise en prise (25) est monté de manière pivotante sur le corps (15) de manière à pouvoir pivoter autour d'un axe de pivotement d'élément de mise en prise.

11. Mécanisme d'ajustement de sangle (10) selon l'une quelconque des revendications précédentes, dans lequel l'élément de mise en prise (25) est allongé et s'étend sur plus de 30 % de la largeur du cliquet (20).

12. Mécanisme d'ajustement de sangle (10) selon l'une quelconque des revendications précédentes, comprenant un élément de sollicitation (38) configuré pour solliciter l'élément de mise en prise (25) jusque dans la position en prise.

13. Mécanisme d'ajustement de sangle (10) selon l'une quelconque des revendications précédentes, comprenant un bouton (28) connecté de manière opérationnelle à l'élément de mise en prise (25), la connexion opérationnelle étant configurée de telle sorte que, lorsqu'il est enfoncé, le bouton (28) provoque le déplacement latéral de l'élément de mise en prise (25) entre le cliquet (20) et le corps (15).

14. Ensemble de sangle, comprenant :
un mécanisme d'ajustement de sangle (10) selon l'une quelconque des revendications précédentes ; et
une sangle (14) s'étendant autour de l'arbre (16) du mécanisme d'ajustement de sangle (10), la sangle (14) comprenant une pluralité d'évidements transversaux espacés le long d'au moins une partie de la longueur de la sangle (14), les évidements étant agencés pour venir en prise avec l'extrémité libre du cliquet (20) du mécanisme d'ajustement de sangle (10).

15. Lunettes (12) comprenant des premier et second oculaires (11) reliés par un pont de nez, au moins l'un des oculaires comprenant un mécanisme d'ajustement (10) selon l'une quelconque des revendications 1 à 13.
